(19) Europäisches Patentamt
European Patent Office
Office européen des brevets

(11) Veröffentlichungsnummer: **0 051 233**
**B1**

(12) # EUROPÄISCHE PATENTSCHRIFT

(45) Veröffentlichungstag der Patentschrift: **08.08.84**

(51) Int. Cl.³: **C 07 D 307/93** // C11B9/00

(21) Anmeldenummer: **81108871.5**

(22) Anmeldetag: **24.10.81**

(54) **Verfahren zur Herstellung von 13-Oxabicyclo(10.3.0)-pentadecan.**

(30) Priorität: **31.10.80 DE 3040994**

(43) Veröffentlichungstag der Anmeldung: **12.05.82 Patentblatt 82/19**

(45) Bekanntmachung des Hinweises auf die Patenterteilung: **08.08.84 Patentblatt 84/32**

(84) Benannte Vertragsstaaten: **AT DE**

(56) Entgegenhaltungen:
**DE - A - 2 810 107**
**FR - A - 1 466 205**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Upadek, Horst, Dr., Kempenweg 18a, D-4006 Erkrath (DE)**
Erfinder: **Bruns, Klaus, Dr., Notburgaweg 6, D-4150 Krefeld-Traar (DE)**

EP 0 051 233 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Gegenstand der Erfindung ist ein verbessertes Verfahren zur Herstellung von 13-Oxabicyclo[10.3.1.]pentadecan.

13-Oxabicyclo[10.3.0.]-pentadecan ist ein wertvoller Riechstoff mit warmer Ambranote und außergewöhnlich guter Haftfestigkeit. Die Herstellung dieses Stoffes ist in DE-A-2 810 107 beschrieben. Das dort aufgeführte Syntheseverfahren geht aus von Cyclododecanon (I), das mit Bromessigester nach Reformatsky zum entsprechenden Hydroxyester (II) umgesetzt wird, der sich im stark sauren Milieu unter Erhitzen leicht in das Lacton (III) umlagert. Dieses Lacton wird mit Natriumborhydrid in Isopropanol zum Diol (IV) reduziert, aus welchem durch intramolekulare Wasserabspaltung in Toluol, in Gegenwart von p-Toluolsulfonsäure, das 13-Oxybicyclo[10.3.0]-pentadecan (V) als Gemisch zweier Stereoisomerer etwa im Molverhältnis 2 : 1 erhalten wird.

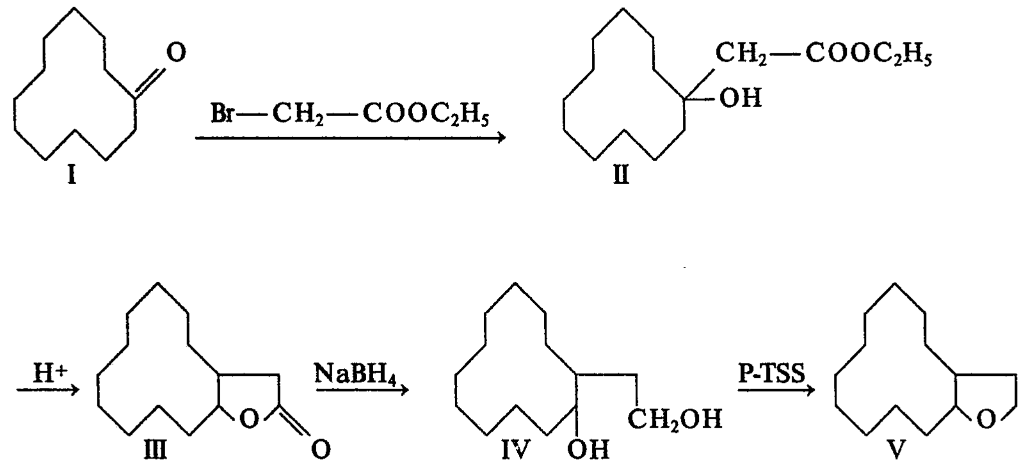


Die Verwendung des sehr toxischen, schleimhautreizenden und flüchtigen Bromessigsäureesters erfordert besondere Sicherheitsmaßnahmen, die nur durch einen entsprechend hohen technischen Aufwand zu realisieren sind. Es bestand daher der Wunsch nach einem Syntheseweg, der sich nicht des Bromessigsäureesters zur Einführung einer Carboxymethylgruppe bedient.

Diese Aufgabenstellung wurde gelöst durch einen neuen Syntheseweg, dessen einzelne Reaktionsschritte literaturbekannte Methoden der präparativen organischen Chemie sind, die aber ausgehend von Cyclododecanon in der erfindungsgemäßen Folge ein neues, technisch wertvolles Verfahren zur Synthese der vielseitig einsetzbaren Riechstoffkomponente 13-Oxabicyclo[10.3.0.]-pentadecan darstellen. Darüber hinaus wurden bei dem erfindungsgemäßen Herstellverfahren für die einzelnen Reaktionsschritte solche Reaktionsbedingungen gefunden, die die Bildung unerwünschter Nebenprodukte weitgehend hintanhalten und die es erlauben, durch einfache, technisch vorteilhafte Reinigungsoperationen die für einen Riechstoff erforderliche Reinheit des Endproduktes zu erzielen.

Das neue Syntheseverfahren bedient sich des Cyclododecanon (I) als Ausgangsverbindung. Durch Bromierung mit elementarem Brom in einem geeigneten organischen Lösungsmittel wird das $\alpha$-Bromcyclododecanon (VI) hergestellt. Dieses wird mit Natrium-malonsäuredialkylester unter streng kontrollierten Reaktionsbedingungen zum 2-(2-Oxocyclododec-1-yl)-malonsäuredialkylester (VII) umgesetzt. Durch Verseifung und Decarboxylierung wird die 2-Oxocyclododec-1-yl-essigsäure (VIII) erhalten, die sich leicht in Form der wäßrigen Lösung ihres Natriumsalzes in alkalischer Lösung durch Auswaschen mit einem organischen Lösungsmittel von neutral reagierenden Nebenprodukten reinigen läßt. Nach Veresterung mit einem niedermolekularen Alkohol erfolgt die Reduktion des Ketoesters (IX) mit einem komplexen Metallhydrid zum 2-(2-Hydroxyethyl)-cyclododecanol (IV). Der letzte Syntheseschritt ist identisch mit dem in DE-A-2 810 107 beschriebenen Verfahren und besteht in der unter Ausbildung des cyclischen Ethers (V) verlaufenden Wasserabspaltung in Toluol in Gegenwart eines sauren Katalysators.

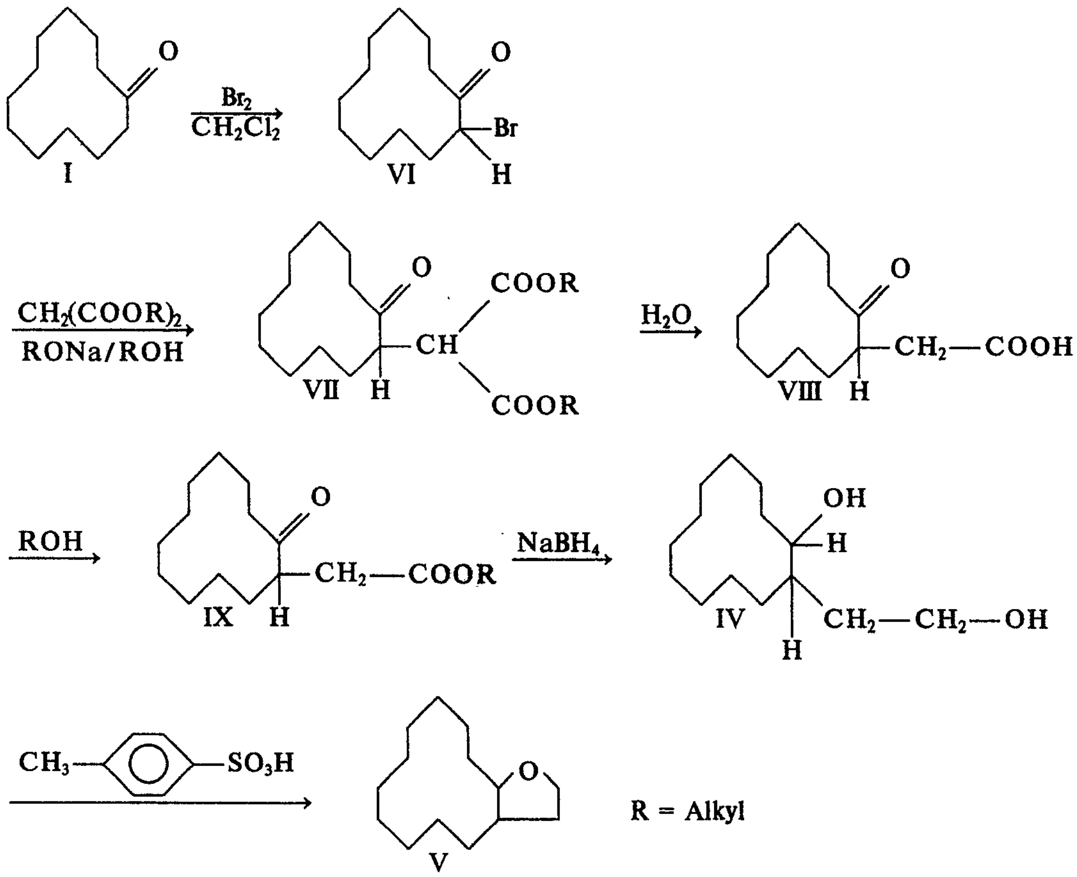


I, VI, VII, VIII, IX, IV, V

R = Alkyl

Für die Bromierung des Cyclododecanon werden in der Literatur zahlreiche Lösungsmittel vorgeschlagen, von welchen die am häufigsten verwendeten — Chloroform und Tetrachlormethan — als human-cancerogene Substanzen in Verdacht und daher für technische Synthesen nicht geeignet sind. Es wurde gefunden, daß die Bromierung zum Beispiel auch in einem Lösungsmittelgemisch aus Eisessig und Acetanhydrid im Molverhältnis 7 : 2 durchgeführt werden kann. Als besonders geeignet zur Erzielung einer hohen Ausbeute von bis zu 90% der Theorie erwies sich die Verwendung von siedendem Methylenchlorid als Lösungsmittel.

Für die Malonester-Synthese kann das rohe $\alpha$-Bromcyclododecanon ohne weitere Reinigung eingesetzt werden. Als Base zur Erzeugung des Malonester-Salzes eignet sich besonders das technisch leicht handhabbare Natriummethylat als 30%ige Lösung im Methanol. Daher verwendet man auch mit Vorteil den Malonsäuredimethylester für diese Umsetzung. Zur Vermeidung der Bildung unerwünschter Nebenprodukte, zum Beispiel durch Favorsky-Umlagerung des $\alpha$-Bromcyclododecanon, erwies es sich als vorteilhaft, dieses rasch unter Kühlung und Einhaltung einer Temperatur von 10—40° C, bevorzugt von 20—30° C zur Umsetzung mit dem Natrium-Malonester zu bringen. Nach Entfernung des Lösungsmittels und Neutralisation mit kalter wäßriger Salzsäure wird das Reaktionsprodukt mit Methylisobutylketon extrahiert.

Die Hydrolyse und Decarboxylierung des Ketodiesters (VII) kann wie üblich durch Verseifung mit verdünnter Natronlauge und anschließendes Erhitzen in schwach saurem, wäßrigem Medium erfolgen. Es wurde jedoch gefunden, daß man ein merklich reineres Reaktionsprodukt erhält, wenn man die Hydrolyse und Decarboxylierung in Gegenwart von Wasser bei 200—250° C im Druckgefäß durchführt und lediglich die dabei nicht verseiften Reste von Ketonmonoester durch Nachverseifung mit verdünnter wäßriger Natronlauge hydrolysiert.

Die so erhaltene wäßrige Lösung des Natriumsalzes der rohen Ketocarbonsäure (VIII) läßt sich vorteilhaft durch Auswaschen mit einem organischen Lösungsmittel, vorzugsweise mit Methylisobutylketon, von neutral reagierenden Nebenprodukten reinigen. Diese Reinigungsoperation ist von entscheidender Wichtigkeit für die Riechstoffqualität des Endprodukts. Nach dem Ansäuern scheidet sich die 2-Oxocyclodec-1-yl-essigsäure (VIII) kristallin in zirka 90%iger Ausbeute ab. Diese Verbindung ist bereits früher nach einem anderen Verfahren, welches in FR-A-1 466 205 (Chimie et Atomistique, 1967) beschrieben ist, in wesentlich geringerer Ausbeute erhalten worden.

Durch Veresterung mit Methanol in Gegenwart eines sauren Katalysators läßt sich die Ketosäure (VIII) nach bekannten Verfahren quantitativ in den Methylester (IX) überführen.

Die Reduktion des Ketoesters (IX) gelingt allgemein mit komplexen Metallhydriden; es wurde jedoch gefunden, daß dem Natriumborhydrid gegenüber anderen Reduktionsmitteln wegen der leichten Handhabung und der selektiv verlaufenden Hydrierung zum 2-(2-Hydroxyethyl)-cyclododecanol

(IV) der Vorzug vor anderen Reduktionsmitteln zu geben ist. Erfindungsgemäß ist zur vollständigen Reduktion des Ketoesters eine Menge von zirka 2 bis 3 Mol Natriumborhydrid pro Mol Ketoester erforderlich. Nach der allgemein üblichen Aufarbeitung wird das Diol (IV) in zirka 85%iger Ausbeute erhalten und ohne weitere Reinigung in einem organischen Lösungsmittel, zum Beispiel in Toluol, der bereits aus der Anmeldung P 2 810 107 bekannten Wasserabspaltung in Gegenwart eines sauren Katalysators, zum Beispiel der p-Toluolsulfonsäure, unter Ausbildung des 13-Oxybicyclo[10.3.0.]-pentadecan (V) unterworfen.

Die im folgenden Beispiel beschriebenen Arbeitsbedingungen sollen das erfindungsgemäße Verfahren näher erläutern ohne es jedoch darauf zu beschränken.

## Beispiel: Herstellung von 13-Oxabicyclo[10.3.0.]-pentadecan

### 1. α-Bromcyclododecanon (VI)

Zu einer gut gerührten Lösung von 182,3 g (1 Mol) Cyclododecanon in 275 ml Methylenchlorid wurden unter Rückfluß in Stickstoffatmosphäre 183,8 g (1,15 Mol) Brom in 6 Stunden zugetropft. Es wurde 15 Minuten nachgerührt und etwa die Hälfte der Lösungsmittelmenge abdestilliert. Die so erhaltene Lösung enthielt zirka 235 g α-Bromcyclododecanon (90% Ausbeute) und wurde direkt weiter umgesetzt.

### 2. 2-(2-Oxocyclododec-1-yl)-malonsäuredimethylester (VII)

Zu 252 g einer 30%igen Natriummethylat/Methanol-Lösung (1,40 Mol Natriummethylat) wurden unter Rühren in Stickstoffatmosphäre 190 g (1,44 Mol) Malonsäuredimethylester so zugetropft, daß die Temperatur nicht über 50°C stieg. Es wurde 1 Stunde bei 40°C gerührt und anschließend bei Raumtemperatur die unter 1. erhaltene Lösung des rohen α-Bromcyclododecanons (ca. 0,9 Mol) in 30 Minuten zum Natriummalonester zugegeben. Die exotherme Reaktion wurde durch Kühlung auf 20—30°C gehalten. Nach beendeter Zugabe wurden Methylenchlorid und Methanol weitgehend entfernt. Der Rückstand wurde mit verdünnter, kalter Salzsäure neutralisiert und mit Methylisobutylketon zweimal extrahiert. Nach Vereinigung der organischen Phasen und Entfernen der flüchtigen Bestandteile unter vermindertem Druck wurde der rohe Keto-Diester (VII) (ca. 250 g; 90% Ausbeute) erhalten und ohne weitere Reinigung zur Verseifung eingesetzt.

### 3. 2-Oxocyclododec-1-yl-essigsäure (VIII)

Der rohe Keto-Diester (ca. 250 g; 0,8 Mol) wurde nach Zusatz von ca. 250 ml Wasser im Autoklaven bei 220°C in 6 Stunden hydrolysiert und gleichzeitig decarboxyliert. Die Mischung wurde mit ca. 1000 ml 5%iger Natronlauge alkalisch gestellt und 2 Stunden bei 40—50°C zur Nachverseifung gerührt. Die wäßrige Phase wurde zur Entfernung neutraler Nebenprodukte einmal mit 1 l Methylisobutylketon gewaschen und anschließend mit konzentrierter Schwefelsäure angesäuert. Die freigesetzte Keto-Monosäure trennte sich als obere Phase ab und wurde nach Ablassen der wäßrigen Phase durch Anlegen von Vakuum bei ca. 80°C getrocknet. Es wurden ca. 175 g kristalline Säure (90% Ausbeute) mit einem Schmelzpunkt von 124°C erhalten.

### 4. 2-Oxocyclododec-1-yl-essigsäuremethylester (IX)

175 g (0,73 Mol) Keto-Monosäure und 440 ml Methanol wurden unter Zusatz von 7,3 g konzentrierter Schwefelsäure 3 Stunden unter Rückfluß gerührt. Die abgekühlte Lösung wurde mit 50%iger Natronlauge neutralisiert und anschließend von Methanol und Wasser vollständig befreit. Der Rückstand enthielt neben geringen Salzmengen (ca. 10 g $Na_2SO_4$) ca. 180 g Keto-Monoester (97% Ausbeute), der direkt weiter umgesetzt wurde.

### 5. 2-(2-Hydroxyethyl)-cyclododecanol (IV)

Der rohe Keto-Ester (ca. 180 g; 0,7 Mol) wurde mit 74 g (1,96 Mol) Natriumborhydrid in 1250 ml Isopropanol 8 Stunden unter Stickstoff-Atmosphäre und Rückfluß gerührt. Nach Zugabe von 150 ml Wasser wurde 30 Minuten bei 60°C nachgerührt und verbliebenes Natriumborhydrid bei guter Kühlung unter Zutropfen von 100 ml Aceton zerstört. Nach einer weiteren halben Stunde Rühren wurde das Isopropanol unter gleichzeitiger Zugabe von 200 ml Wasser abdestilliert. Nach Abkühlen auf ca. 30—35°C wurde der Rückstand mit halbkonzentrierter Schwefelsäure neutralisiert und mit 700 ml

Toluol versetzt. Die wäßrige Phase wurde abgetrennt. Die Toluolphase enthielt ca. 135 g 1,4-Diol (85% Ausbeute) und wurde ohne Einengung zur Dehydratisierung eingesetzt.

6. 13-Oxabicyclo[10.3.0.]-pentadecan (V)

Die unter 5. erhaltene Toluol-Lösung des Diols (ca. 0,59 Mol) wurde mit 12 g p-Toluolsulfonsäure versetzt und unter Rühren ca. 4 Stunden am Wasserabscheider erhitzt. Nach beendeter Reaktion wurde mit 5%iger Natronlauge neutralisiert, die wäßrige Phase abgetrennt und die Toluolphase eingeengt. Durch Vakuumdestillation wurden bei 90—95° C/0,25 mbar ca. 80 g 13-Oxabicyclo[10.3.0.]-pentadecan (65% Ausbeute) isoliert.

**Patentansprüche**

1. Verbessertes Verfahren zur Herstellung von 13-Oxabicyclo[10.3.0.]-pentadecan aus Cyclododecanon, gekennzeichnet durch die Reaktionsfolge a—g:

(a) Umsetzung von Cyclododecanon mit elementarem Brom in einem organischen Lösungsmittel,
(b) Umsetzung des α-Bromcyclododecanon mit Malonsäuredialkylester und Alkalimetallalkoholat zum 2-(2-Oxocyclododec-1-yl)malonsäuredialkylester,
(c) Hydrolyse und Decarboxylierung des Keto-Diesters zur 2-Oxocyclododec-1-yl-essigsäure,
(d) Reinigung der rohen 2-Oxocyclododec-1-yl-essigsäure durch Auswaschen der wäßrig-alkalischen Lösung mit einem organischen Lösungsmittel,
(e) Veresterung mit einem niedermolekularen Alkohol, zum Beispiel Methanol,
(f) Reduktion des Ketoesters mit einem komplexen Metallhydrid zum 2-(2-Hydroxyethyl)-cyclododecanol und
(g) cyclische Etherbildung in Gegenwart eines sauren Katalysators.

2. Verfahren nach Anspruch 1, dadurch gekennzeichnet, daß die Bromierung von Cyclododecanon in siedendem Methylenchlorid als Lösungsmittel durchgeführt wird.

3. Verfahren nach Anspruch 1, dadurch gekennzeichnet, daß das rohe α-Bromcyclododecanon bei 10—40° C mit Natrium-Malonsäuredimethylester in Methanol umgesetzt wird.

4. Verfahren nach Anspruch 1, dadurch gekennzeichnet, daß die Hydrolyse und Decarboxylierung in einem Druckgefäß bei 200—250° C mit verdünnter Natronlauge in Gegenwart von Wasser durchgeführt wird.

5. Verfahren nach Anspruch 1, dadurch gekennzeichnet, daß rohe 2-Oxocyclodec-1-yl-essigsäure in Form der wäßrigen, alkalischen Lösung des Alkalisalzes, gegebenenfalls nach kurzer Nachverseifung, zur Entfernung neutral reagierender Nebenprodukte mit Methylisobutylketon ausgewaschen wird.

6. Verfahren nach Anspruch 1, dadurch gekennzeichnet, daß der rohe 2-Oxocyclododec-1-yl-essigsäureester mit 2,0 bis 3,0 Mol Natriumborhydrid pro Mol Ketoester in Isopropanol als Lösungsmittel und unter Stickstoff als Schutzgas reduziert wird.

**Claims**

1. An improved process for the production of 13-oxabicyclo-[10.3.0]-pentadecane from cyclododecanone, characterized by the following reaction sequence a—g:

(a) reacting cyclododecanone with elemental bromine in an organic solvent,
(b) reacting the α-bromocyclododecanone with malonic acid dialkyl ester and an alkali metal alcoholate to form 2-(2-oxocyclododec-1-yl-malonic acid dialkyl ester,
(c) hydrolyzing and decarboxylating the keto diester to form 2-oxocyclododec-1-yl acetic acid,
(d) purifying the crude 2-oxocyclododec-1-yl acetic acid by washing out the aqueous-alkaline solution with an organic solvent,
(e) esterification with a low molecular weight alcohol, for example methanol,
(f) reducing the keto ester with a complex metal hydride to form 2-(2-hydroxyethyl)-cyclododecanol and
(g) formation of the cyclic ether in the presence of an acidic catalyst.

2. A process as claimed in claim 1, characterized in that the bromination of cyclododecanone is carried out in boiling methylene chloride as solvent.

3. A process as claimed in claim 1, characterized in that the crude α-bromocyclododecanone is reacted with sodium malonic acid dimethyl ester in methanol at 10 to 40° C.

4. A process as claimed in claim 1, characterized in that the hydrolysis and decarboxylation reactions are carried out in the presence of water in a pressure vessel at 200 to 250° C using dilute sodium hydroxide.

5. A process as claimed in claim 1, characterized in that the crude 2-oxocyclodec-1-yl acetic acid in the form of an aqueous, alkaline solution of the alkali salt is washed with methyl isobutyl ketone to remove neutrally reacting secondary products, optionally after brief further hydrolysis.

6. A process as claimed in claim 1, characterized in that the crude 2-oxocyclododec-1-yl acetic acid ester is reduced with from 2.0 to 3.0 moles of sodium borohydride per mole of keto ester in isopropanol as solvent and under nitrogen as inert gas.

**Revendications**

1. Procédé amélioré de fabrication de 13-oxabicyclo[10.3.0]-pentadécane à partir de cyclododécanone, caractérisé par la série de réactions a—g:

(a) réaction de la cyclododécanone avec du brome élémentaire dans un solvant organique,
(b) réaction de l'α-bromocyclododécanone avec un malonate de dialcoyle et un alcoolate de métal alcalin en ester dialcoylé d'acide 2-(2-oxocyclododéc-1-yl)-malonique,
(c) hydrolyse et décarboxylation du céto-diester en acide 2-oxocyclododéc-1-yl-acétique,
(d) purification de l'acide 2-oxocyclododéc-1-yl-acétique brut par lavage extractif de la solution alcaline aqueuse avec un solvant organique,
(e) estérification avec un alcool à poids moléculaire inférieur, par exemple du méthanol,
(f) réduction du céto-ester avec un hydrure métallique complexe en 2-(2-hydroxyéthyl)-cyclododécanol et
(g) formation d'éther cyclique en présence d'un catalyseur acide.

2. Procédé selon la revendication 1, caractérisé en ce qu'on exécute la bromuration de la cyclododécanone dans du chlorure de méthylène bouillant comme solvant.

3. Procédé selon la revendication 1, caractérisé en ce que l'α-bromocyclododécanone brute est mise à réagir à 10—40° C avec du malonate de diméthylsodium dans du méthanol.

4. Procédé selon la revendication 1, caractérisé en ce qu'on effectue l'hydrolyse et la décarboxylation dans un récipient à pression à 200—250° C avec de la soude caustique diluée en présence d'eau.

5. Procédé selon la revendication 1, caractérisé en ce que l'acide 2-oxocyclododéc-1-yl-acétique sous forme de la solution alcaline aqueuse du sel alcalin, éventuellement après une post-saponification de courte durée, est épuisé par lavage avec de la méthylisobutylcétone pour éliminer les sous-produits à réaction neutre.

6. Procédé selon la revendication 1, caractérisé en ce que l'ester d'acide 2-oxocyclododéc-1-yl-acétique brut est réduit avec 2,0 à 3,0 moles de borohydrure de sodium par mole de céto-ester dans de l'isopropanol comme solvant et sous azote comme gaz protecteur.